# EUROPEAN PATENT SPECIFICATION

(11) **EP 2 712 318 B1**
(45) Date of publication and mention of the grant of the patent: **14.03.2018**
(21) Application number: 12722835.1
(22) Date of filing: 18.04.2012
(51) Int. Cl.: A61K 35/74, C12R 1/25, C12R 1/23, C12R 1/46, A61K 35/747, C12N 1/20, C12R 1/01, A23L 33/135, A61K 35/745, A61K 45/06

(54) **COMPOSITION COMPRISING PROBIOTIC BACTERIA CAPABLE OF RESTORING THE BARRIER EFFECT OF THE STOMACH WHICH IS LOST DURING PHARMALOGICAL TREATMENT OF GASTRIC HYPERACIDITY**
ZUSAMMENSETZUNG MIT PROBIOTISCHEN BAKTERIEN ZUR WIEDERHERSTELLUNG DER WÄHREND EINER PHARMAKOLOGISCHEN BEHANDLUNG VON MAGENÜBERSÄUERUNG VERLORENGEGANGENEN BARRIEREWIRKUNG DES MAGENS
COMPOSITION COMPRENANT DES BACTÉRIES PROBIOTIQUES APTE À RESTAURER L'EFFET BARRIÈRE DE L'ESTOMAC QUI EST PERDU LORS D'UN TRAITEMENT PHARMACOLOGIQUE DE L'HYPERACIDITÉ GASTRIQUE

(30) Priority: 20.04.2011 IT MI20110679
(43) Date of publication of application: 02.04.2014
(73) Proprietor: Probiotical S.p.A., 28100 Novara (IT)
(72) Inventor: MOGNA, Giovanni, 28100 Novara (IT)
(74) Representative: Hoffmann Eitle
(86) International application number: PCT/IB2012/000779
(87) International publication number: WO 2012/143787

(56) References cited:
- VASILJEVIC T ET AL: "Probiotics-From Metchnikoff to bioactives", INTERNATIONAL DAIRY JOURNAL, ELSEVIER APPLIED SCIENCE, BARKING, GB, vol. 18, no. 7, 1 July 2008 (2008-07-01), pages 714-728, XP022701025, ISSN: 0958-6946, DOI: 10.1016/J.IDAIRYJ.2008.03.004 [retrieved on 2008-03-07]
- HAMILTON-MILLER J M T: "The role of probiotics in the treatment and prevention of Helicobacter pylori infection.", INTERNATIONAL JOURNAL OF ANTIMICROBIAL AGENTS OCT 2003 LNKD- PUBMED:14522098, vol. 22, no. 4, October 2003 (2003-10), pages 360-366, XP002661415, ISSN: 0924-8579
- WANG KUAN-YUAN ET AL: "Effects of ingesting Lactobacillus- and Bifidobacterium-containing yogurt in subjects-with colonized Helicobacter pylori", THE AMERICAN JOURNAL OF CLINICAL NUTRITION, AMERICAN SOCIETY FOR NUTRITION, US, vol. 80, no. 3, 1 September 2004 (2004-09-01), pages 737-741, XP002490404, ISSN: 0002-9165
- SGOURAS DIONYSSIOS N ET AL: "Lactobacillus johnsonii La1 attenuates Helicobacter pylori-associated gastritis and reduces levels of proinflammatory chemokines in C57BL/6 mice", CLINICAL AND DIAGNOSTIC LABORATORY IMMUNOLOGY, AMERICAN SOCIETY FOR MICROBIOLOGY, US, vol. 12, no. 12, 1 December 2005 (2005-12-01), pages 1378-1386, XP002606907, ISSN: 1071-412X, DOI: 10.1128/CDLI.12.12.1378-1386.2005

## Description

The present invention relates to a composition comprising probiotic bacteria for use in the pharmacological treatment of gastric hyperacidity. Said composition is capable of restoring the barrier effect of the stomach which is lost during pharmacological treatment of gastric hyperacidity and of minimizing the secondary effects due to said pharmacological treatment.

Over the last decades various pharmacological approaches have been developed for pharmacologically treating gastric hyperacidity, a condition that, if present in an accentuated manner and for a long time, can give rise to various complications or pathologies such as peptic ulcer and gastroesophageal reflux disease.

Among the most widely used drugs there are those based on active ingredients capable of inhibiting histamine H₂ receptor inhibitors such as, for example, cimetidine, famotidine, nizatidine and ranitidine or based on active ingredients capable of inhibiting prostaglandins such as, for example, misoprostol.

Another category of drugs is based on active ingredients that perform a function of gastric mucosa protectors such as, for example, bismuth salts, sucralfate or antimuscarinic or parasympatholytic drugs based on pirenzepine and pipenzolate. Finally, there are also antacids such as, for example, sodium bicarbonate, aluminium hydroxide or magnesium hydroxide and proton pump inhibitors based on Lansoprazole, Esomeprazole, Rabeprazole, Pantoprazole and Omeprazole.

Proton pump inhibitors (PPI) are a group of molecules whose principal action is represented by a pronounced reduction of the acidity of gastric juices for a fairly long period of time (from 18 to 24 hours).

The group containing PPIs is the successor of the H₂ antihistamines and PPI inhibitors are much more widespread than the latter given their greater effectiveness.

The above-mentioned medicines are used in the symptomatic and etiological treatment of various syndromes, such as (i) dyspepsia; and (ii) gastroduodenal ulcer. PPIs are used to treat or prevent gastric and duodenal ulcers. They are also used in association with several antibiotics in the treatment of gastritis caused by *Helicobacter pylori;* (iii) Zollinger-Ellison syndrome and (iv) gastroesophageal reflux disease.

PPIs are also used in patients treated with acetyl salicylic acid or other long-term NSAIDs. Such drugs, by inhibiting the function of the enzyme cyclooxygenase 1 (COX 1), have a side effect of reducing prostaglandin synthesis, a process that depends on that very enzyme.

Since the functions of prostaglandins include protecting the gastric mucosa from acidity, PPIs are used to reduce acidity and protect the gastric mucosa.

This type of medicine inhibits the gastric enzyme H⁺/K⁺-ATPase (proton pump), catalyser of the exchange of H⁺ and K⁺ ions. This creates an effective inhibition of acid secretion.

In the microchannel where the pH is low, close to 2, these inhibitors are ionized and transformed into molecules capable of establishing covalent bonds with the thiol group (SH) of cysteine of the subunit of the pump. Thus the pump is irreversibly inhibited. The resumption of pumping activity entails the production of new pumps, an event which requires 18 to 24 hours on average. A single dose of PPIs therefore enables gastric secretion to be inhibited for about 24 hours.

The fact that the inhibitors are active only in an acidic environment explains why they have a minimal effect on the extra-gastric H⁺/K⁺-ATPase situated at the level of the rectum and colon.

In any case, above and beyond their specific mechanism of action, the final effect of nearly all of these classes of drugs for treating gastric hyperacidity or the other pathological conditions mentioned above is to increase the gastric pH, with kinetics and intensities that depend on the specific pharmacological molecule taken and the dosage thereof. Exceptions in this respect are prostaglandins and gastric mucosa protector drugs, which, instead of reducing the intraluminal hydrogen ion concentration, increase the synthesis of mucus and bicarbonate ions by the cells of the gastric wall, thereby increasing the protection of the mucosa against the acidity of the lumen. In any case, the drugs capable of reducing gastric hyperacidity constitute the treatment of choice in the case of peptic ulcers or gastroesophageal reflux, whereas mucosa protectors represent a complementary therapy.

It is well known, though, that a normal gastric acidity constitutes an effective barrier against potentially harmful or pathogenic microorganisms ingested in a normal diet. Many of them, in fact, are particularly sensitive to acidity and are not able to survive for more than 5 minutes, sometimes even less, at pH values lower than 3. It follows that many pathogens, including those belonging to the genus *Salmonella,* do not reach the intestine alive and, unless there are harmful effects on the human body mediated by secreted toxins already present in the food, they are not capable of giving rise to an intestinal infection and hence a full-blown food intoxication.

It should be said, however, that raising the gastric pH values typically found in subjects who take drugs to reduce or treat gastric hyperacidity renders the same subjects more exposed to risks of food toxinfections caused above all by the consumption of raw foods, especially fish, meat and eggs.

Subjects who take drugs such as, for example, proton pump inhibitors, to reduce or treat gastric hyperacidity, exhibit a stomach pH value of around 5.

This pH value allows Enterobacteriaceae and particular strains of *E. Coli,* endowed with marked decarboxylase activity, to pass intact through the degraded gastric barrier. The proteins ingested in the diet are enzymatically degraded to amino acids, which, in the presence of decarboxylase activity, are modified into a series of biogenic amines such as, for example histamine, tyramine, putrescine and cadaverine, which range from potentially dangerous to very dangerous. The most common symptoms that these biogenic amines can cause are wholly similar to the secondary effects caused by the use of proton pump inhibitors (PPI) and they are: diarrhoea, headache, nausea, abdominal pains and flatulence. Moreover, when certain biogenic amines react with nitrites we have the formation of N-nitrosamines. These nitrosamines provoke a genetic mutation by alkylating DNA and their presence is associated with stomach, intestinal, pancreatic and bladder cancer as well as leukaemia.

Suspending the pharmacological therapy obviously does not represent a possible solution for these subjects, since that would once again expose the gastric or esophageal mucosa to the harmful effects mediated by gastric juices. On the other hand, it is unthinkable to continue the pharmacological therapy and leave the subjects exposed to such risks of infection.

Thus, there remains a necessity to enable subjects in need to take drugs to reduce or treat gastric hyperacidity, on the one hand, and to avoid being exposed to highly dangerous pathogenic infections or to risks of relapsing pathogenic infections, on the other hand.

The Applicant has provided an answer to the above-mentioned necessity thanks to a composition capable of restoring the functionality of the gastric barrier which has a protective effect against pathogenic or harmful microorganisms.

The composition of the present invention is capable of restoring the gastric barrier function which is normally performed by gastric juices and is particularly reduced in subjects who take drugs to reduce or treat gastric hyperacidity. Said composition is capable of minimizing the secondary effects associated with a pharmacological therapy based on proton pump inhibitor drugs (PPIs for short).

The composition of the present invention can be validly used in the treatment of peptic ulcer or gastroesophageal reflux.

After intense research activity, the Applicant surprisingly found that a selected combination of probiotic bacteria is capable of enabling subjects in need to take drugs to reduce or treat gastric hyperacidity, on the one hand, and to avoid being exposed to highly dangerous pathogenic infections or to risks of relapsing pathogenic infections, on the other hand.

Therefore, the subject matter of the present invention is a composition having the features described in the appended independent claim.

Other preferred embodiments of the present invention are described hereunder and will be claimed in the appended dependent claims.

Table 1 shows, by way of example, a group of microorganisms which has valid application in the context of the present invention.

The Applicant conducted an intense activity of research and selection, at the end of which it found that the probiotic bacterial strains belonging to the group:
*1. Lactobacillus pentosus* LPS01 DSM 21980
*2. Lactobacillus plantarum* LP01 LMG P-21021
3. *Lactobacillus plantarum* LP02 LMG P-21020
4. *Lactobacillus plantarum* LP03 LMG P-21022
5. *Lactobacillus plantarum* LP04 LMG P-21023
6. *Lactobacillus rhamnosus* LR06 DSM 21981
7. *Lactobacillus delbrueckii* LDD 01 (MB386) DSMZ 20074 DSM 22106.
*8. Bifidobacterium longum* B1975 DSM 24709
*9. Bifidobacterium breve* B2274 DSM 24707
10. *Bifidobacterium breve* B632 DSM 24706
11. *Bifidobacterium breve* B7840 DSM 24708
*12. Bifidobacterium longum* PCB 133 DSM 24691
13. *Bifidobacterium longum* BL06 DSM 24689,
have valid application in the treatment of subjects who take drugs to reduce or treat gastric hyperacidity.

The bacterial strains were selected because they are capable of colonizing the stomach at a pH value comprised between 4.5 and 5. At this pH value the selected strains act by producing active substances such as bacteriocins and/or metabolites and/or hydrogen peroxide.

The composition for use according to the present invention can be a food composition, for example a symbiotic composition, or else a supplement or a pharmaceutical composition or a medical device.

In an aspect, the composition can comprise one or more strains selected from among those listed in Table 1.

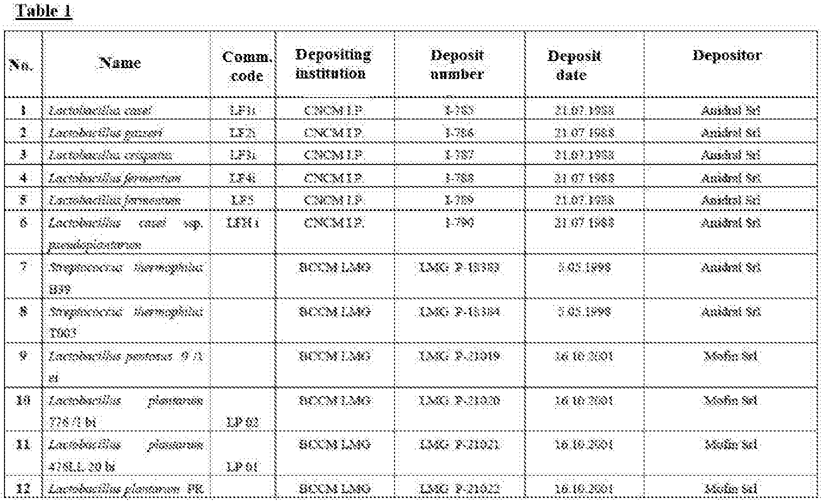

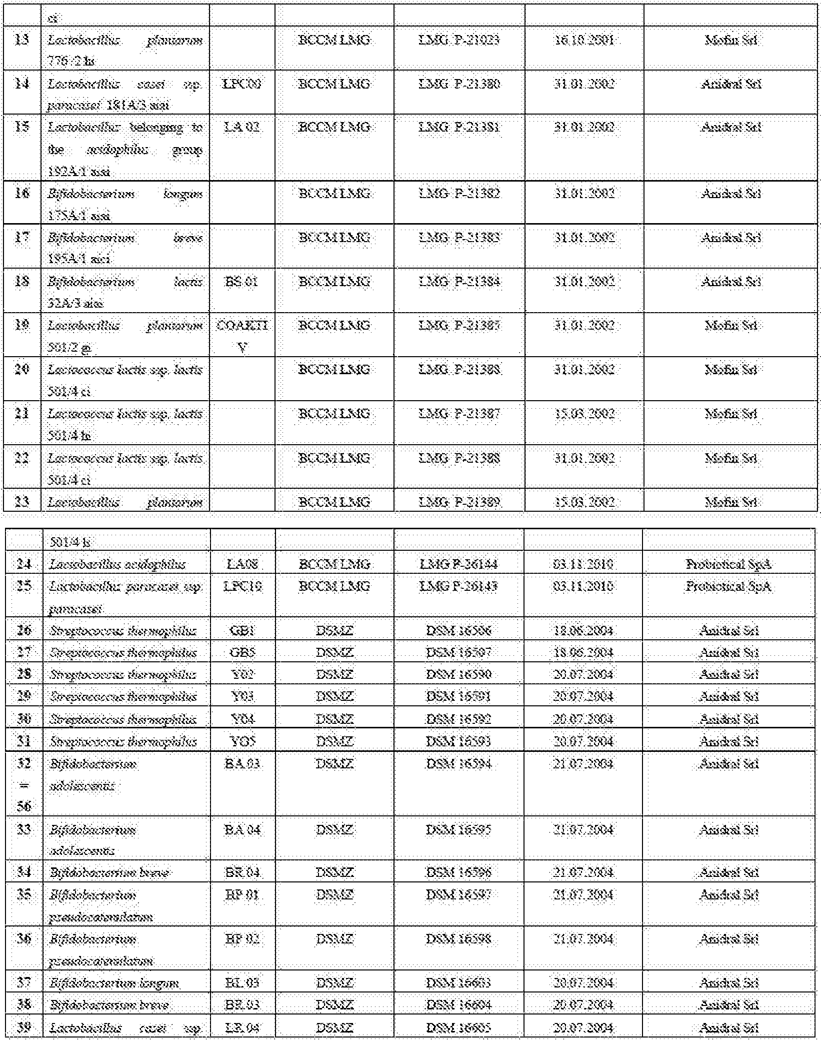

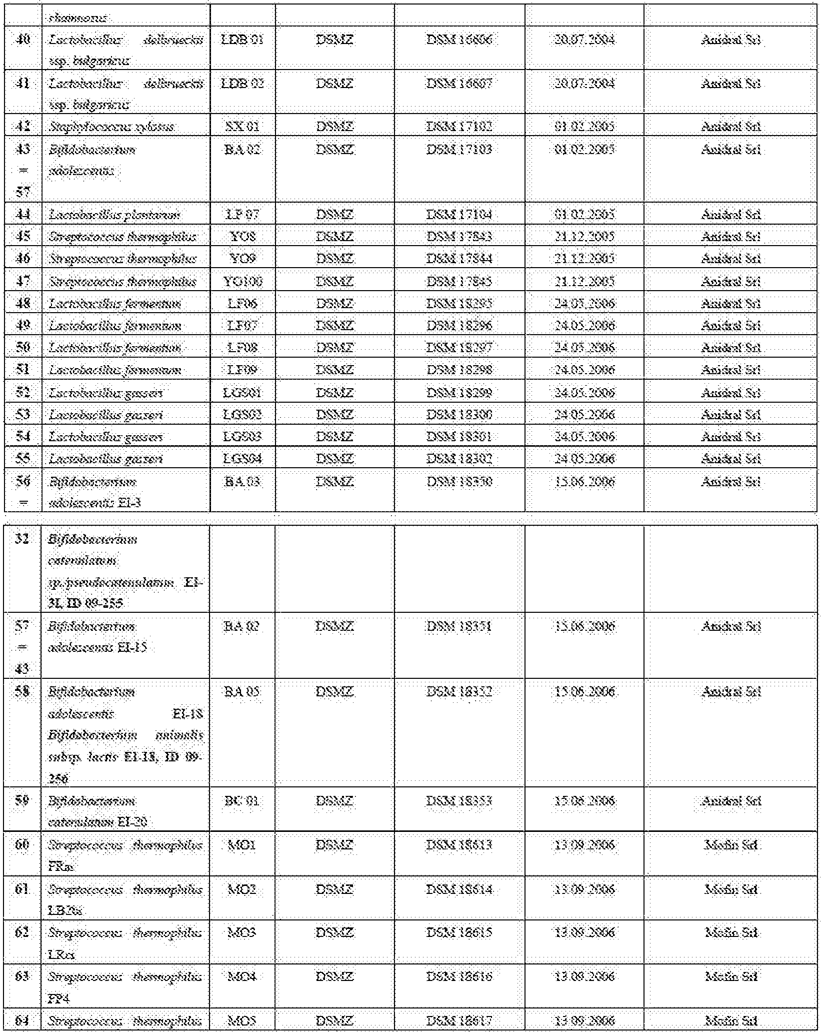

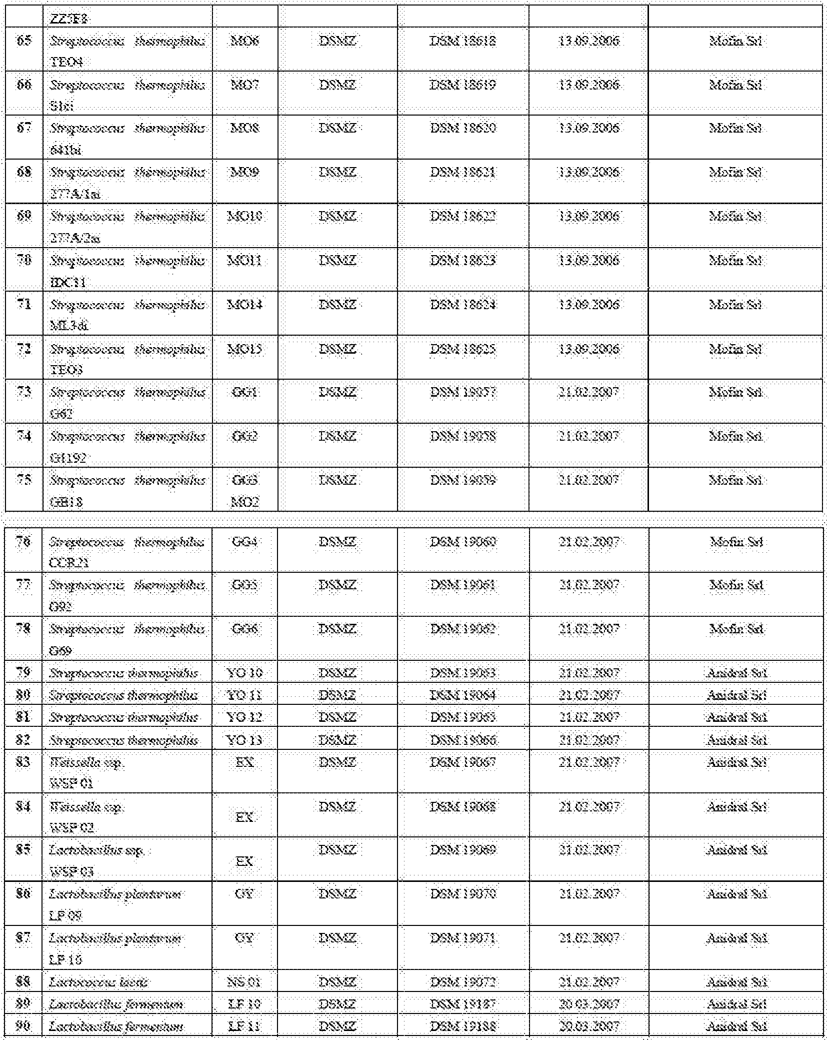

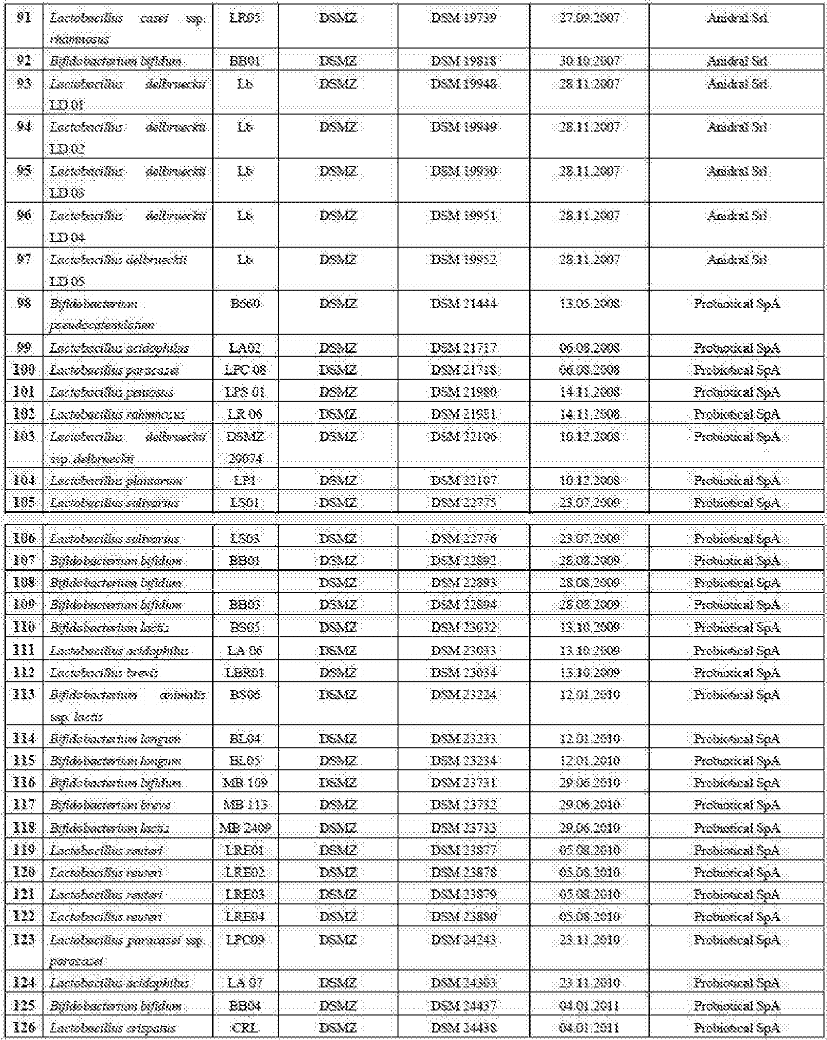

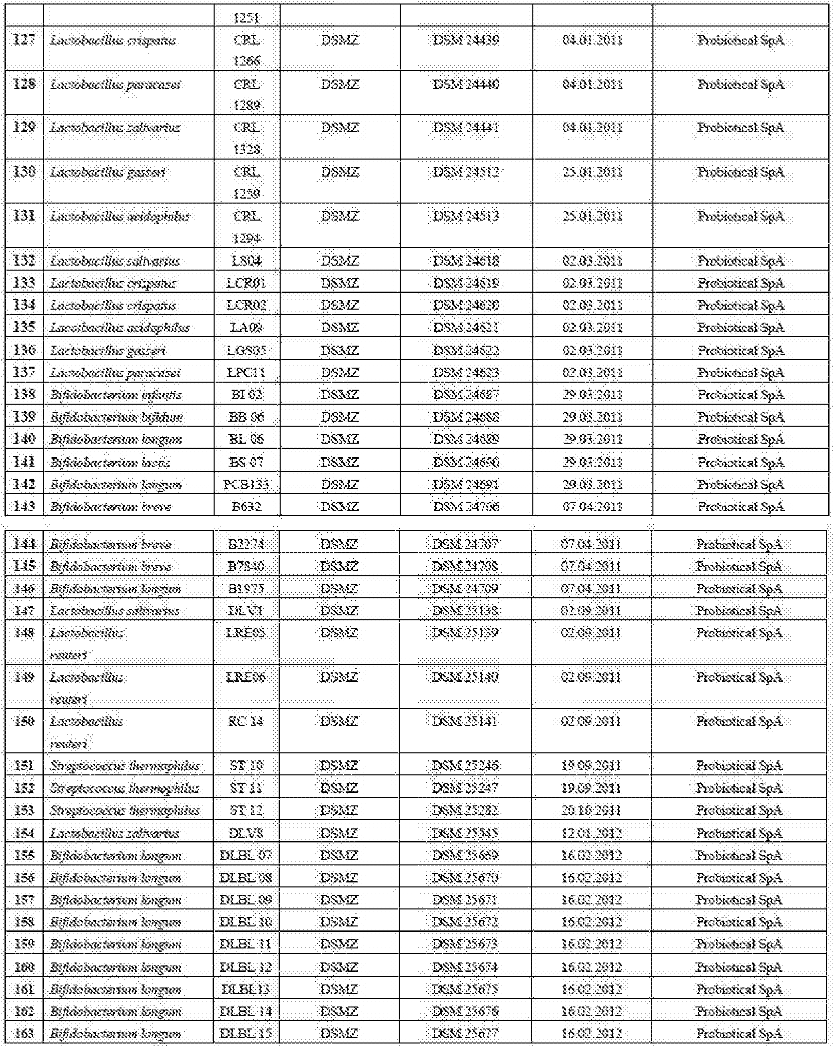

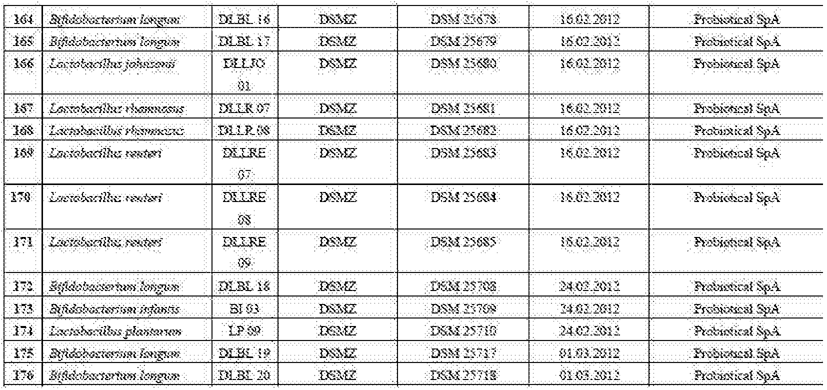

In an aspect, the composition comprises strains selected from among those listed in Table 2.

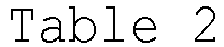

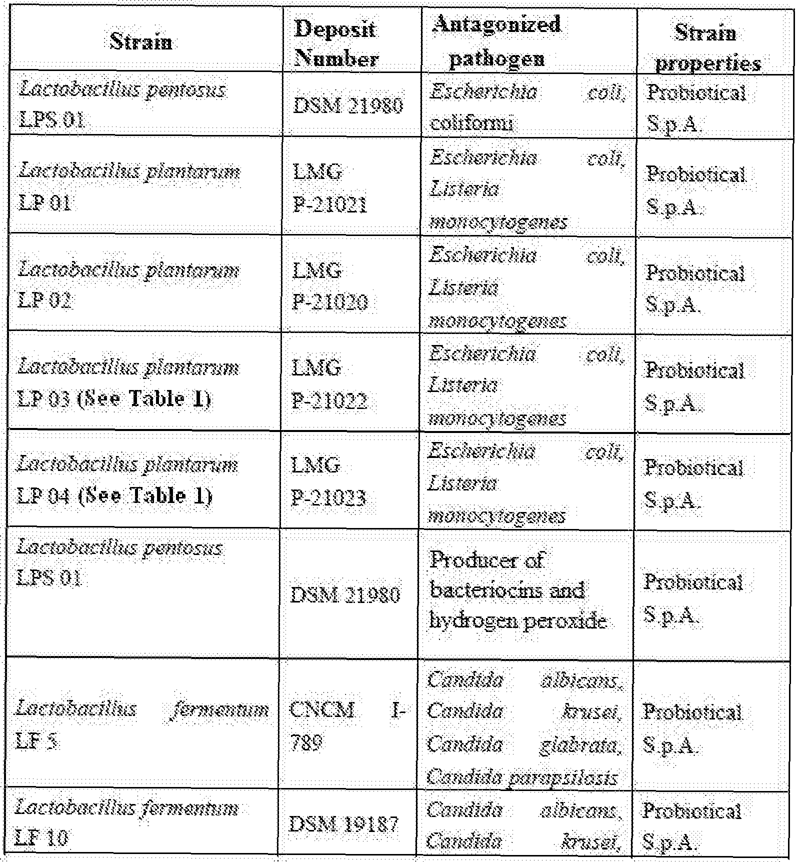

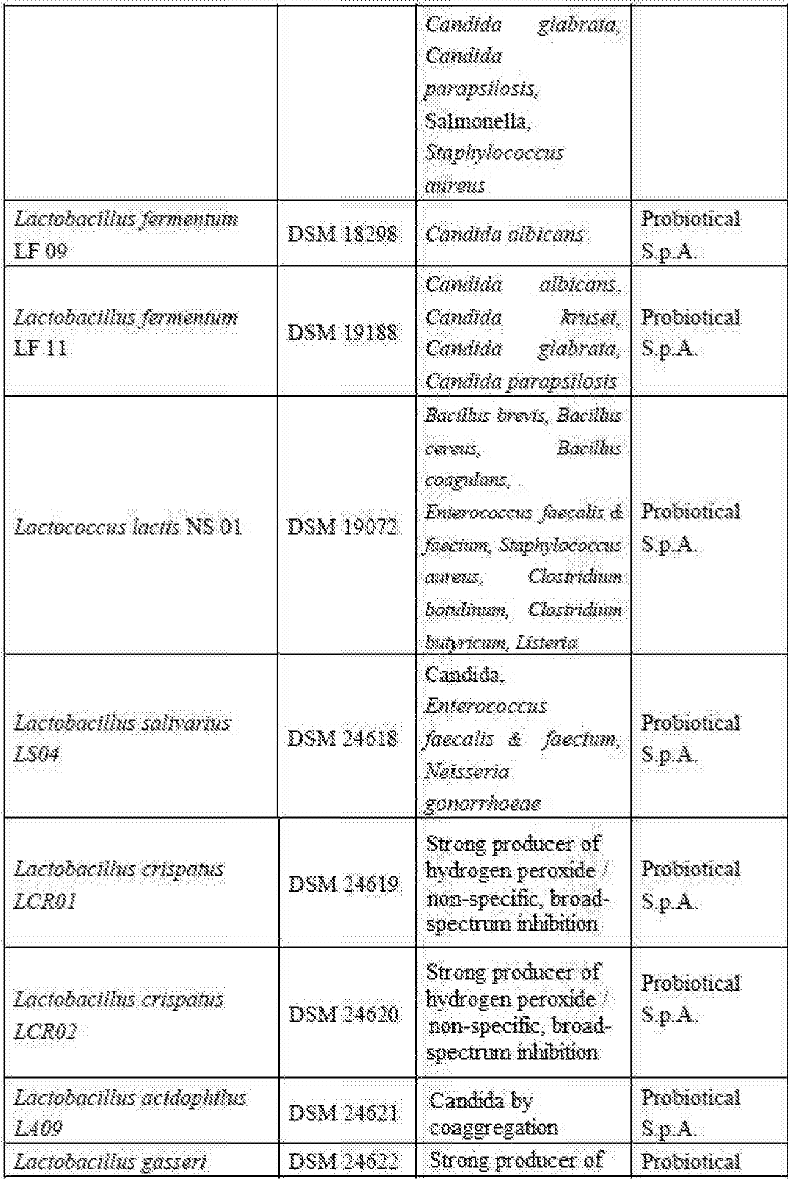

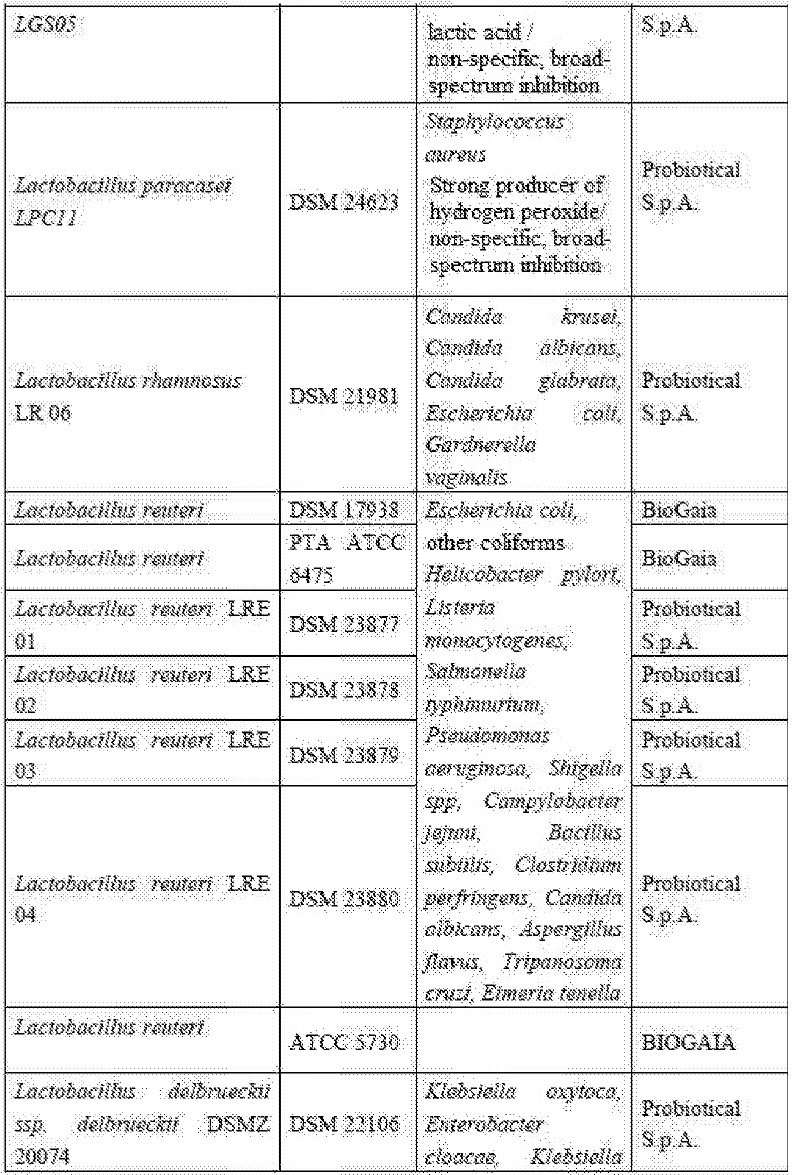

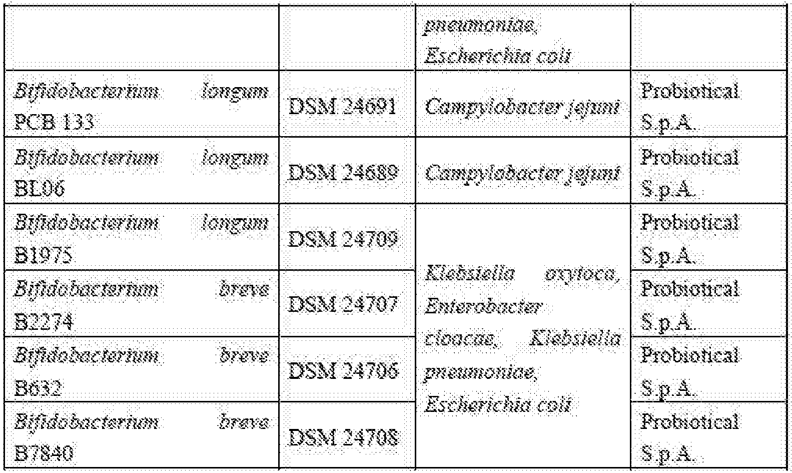

The strains in Table 2 were individually tested in order to identify the pathogen they are capable of antagonizing (by inhibiting growth or reducing the number of one or more harmful or pathogenic microbial species/genera), as shown in column 3 of Table 2.

Table 2 shows that the bacteria are capable of producing hydrogen peroxide or at least one bacteriocin with an inhibiting activity on one or more harmful or pathogenic microbial species/genera.

All of the strains described and/or claimed in the present patent application have been deposited in accordance with the Budapest Treaty and are made available to the public, on request, by the competent Depositing Authority.

The compositions of the present invention have valid application for use both in the treatment of subjects who take drugs to reduce or treat gastric hyperacidity and in the treatment of an ulcer caused by a deficiency in the protective mechanisms of the mucosa (e.g. reduced secretion or responsiveness to prostaglandin E, as in the case of the intake of aspirin or other NSAIDs) or an *H. pylori* infection. In other words, the composition of the present invention has application also in subjects whom are prescribed PPIs/other antacid drugs though they do not have gastric hyperacidity, but rather a lesion of the gastric and/or duodenal mucosa resulting from an altered ratio between gastric acidity and the protector mechanisms of the mucosa.

The composition for use according to the invention comprises at least four strains selected from the group comprising or, alternatively, consisting of:
*1. Lactobacillus pentosus* LPS01 DSM 21980
*2. Lactobacillus plantarum* LP01 LMG P-21021
3. *Lactobacillus plantarum* LP02 LMG P-21020
4. *Lactobacillus plantarum* LP03 LMG P-21022
5. *Lactobacillus plantarum* LP04 LMG P-21023
6. *Lactobacillus rhamnosus* LR06 DSM 21981
7. *Lactobacillus delbrueckii* LDD 01 (MB386) DSM 20074
8. *Bifidobacterium longum* B1975 DSM 24709
9. *Bifidobacterium breve* B2274 DSM 24707
10. *Bifidobacterium breve* B632 DSM 24706
11. *Bifidobacterium breve* B7840 DSM 24708
*12. Bifidobacterium longum* PCB 133 DSM 24691
13. *Bifidobacterium longum* BL06 DSM 24689

In another preferred embodiment, the composition for use according to the present invention comprises at least four strains selected from those indicated above as 1 to 13, in association with the strain *Lactobacillus fermentum* LF 09 DSM 18298 and/or the strain *Lactococcus lactis* NS 01 DSM 19072.

In another preferred embodiment, the composition for use according to the present invention comprises at least four strains, selected from those indicated above as 1 to 13, in association with at least one strain selected from the group comprising or, alternatively, consisting of:
a. *Lactobacillus reuteri* LRE 01 DSM 23877
b. *Lactobacillus reuteri* LRE 02 DSM 23878
c. *Lactobacillus reuteri* LRE 03 DSM 23879
d. *Lactobacillus reuteri* LRE 04 DSM 23880

In the composition of the present invention, the mixture of bacterial strains is present in an amount comprised from 0.5 to 20% by weight, relative to the total weight of the composition, preferably from 2.5 to 8%.

In a preferred embodiment, the composition can further comprise at least one prebiotic fibre and/or carbohydrates with bifidogenic action.

The prebiotic fibre that has application in the composition of the present invention is a fibre that must be used by the bacterial strains present in the composition, but not by the pathogens they are intended to antagonize.

If the pathogen to be antagonized belongs to the genus *Candida,* fructo-oligosaccharides (FOS) and galacto-oligosaccharides (GOS) have valid application, since said fibres are not utilized by Candida. Whereas, gluco-oligosaccharides (GOSα) are capable of directly inhibiting *E. coli* by means of several metabolites.

Therefore, the prebiotic fibre can be selected, depending on circumstances and the pathogen to be antagonized, from among: inulin, fructo-oligosaccharides (FOS), galacto- and trans-galacto-oligosaccharides (GOS and TOS), gluco-oligosaccharides (GOSα), xylo-oligosaccharides (XOS), chitosan oligosaccharides (COS), soy oligosaccharides (SOS), isomalto-oligosaccharides (IMOS), resistant starch, pectin, psyllium, arabinogalactans, glucomannans, galactomannans, xylans, lactosucrose, lactulose, lactitol and various other types of gums, acacia, carob, oat or bamboo fibre, citrus fibres and, in general, fibres containing a soluble and an insoluble portion, in a variable ratio to each other.

In a preferred embodiment of the invention, the composition comprises at least one prebiotic fibre selected from among the ones mentioned above and/or suitable mixtures thereof in any relative percentage.

The amount of the prebiotic fibres and/or carbohydrates having a bifidogenic action, if present in the composition, is comprised from 0 to 60% by weight, preferably from 5 to 45% and even more preferably from 10 to 30%, relative to the total weight of the composition. In this case the composition or supplement has symbiotic activity and functional properties. Moreover, the composition can further comprise other active ingredients and/or components, such as vitamins, minerals, bioactive peptides, substances having antioxidant, hypocholesterolemizing, hypoglycemizing, anti-inflammatory or anti-sweetener activity in an amount by weight generally comprised from 0.001% to 20% by weight, preferably from 0.01% to 5%, depending in any case on the type of active component and the recommended daily dose thereof, if defined, relative to the total weight of the composition.

The food composition of the present invention, for example a symbiotic composition, or a supplement or a pharmaceutical composition, is prepared using techniques and equipment known to a person skilled in the art.

In a preferred embodiment, the composition contains bacteria in a concentration comprised from 1x10⁶ to 1x10¹¹ CFU/g of mixture, preferably from 1x10⁸ to 1x10¹⁰ CFU/g of mixture.

In a preferred embodiment, the composition contains bacteria in a concentration comprised from 1x10⁶ to 1x10¹¹ CFU/dose, preferably from 1x10⁸ to 1x10¹⁰ CFU/dose.

The dose can be comprised from 0.2 to 10 g; for example it is 0.25 g, 1 g, 3 g, 5 g or 7 g.

The probiotic bacteria used in the present invention can be in solid form, in particular in powder, dehydrated powder or lyophilized form.

All of the compositions of the present invention are prepared according to techniques known to a person skilled in the art and using known equipment.

In a preferred embodiment, the composition of the present invention further comprises a drug to reduce or treat gastric hyperacidity.

In a preferred embodiment, said drug is selected from the group comprising or, alternatively, consisting of:
- H2 receptor inhibitors, preferably cimetidine, famotidine, nizatidine or ranitidine;
- prostaglandins, preferably misoprostol;
- gastric mucosa protectors, preferably bismuth salts or sucralfate;
- muscarinic antagonists or parasympatholytic agents, preferably pirenzepine or pipenzolate;
- antacids, preferably sodium bicarbonate, aluminium hydroxide, magnesium hydroxide;
- proton pump inhibitors, preferably Lansoprazole, Esomeprazole, Rabeprazole, Pantoprazole and Omeprazole.

Preferably, said drug is selected from the group comprising or, alternatively, consisting of:
- H2 receptor inhibitors, preferably cimetidine, famotidine, nizatidine or ranitidine;
- muscarinic antagonists or parasympatholytic agents, preferably pirenzepine or pipenzolate;
- antacids, preferably sodium bicarbonate, aluminium hydroxide, magnesium hydroxide;
- proton pump inhibitors, preferably selected from the group comprising Lansoprazole, Esomeprazole, Rabeprazole, Pantoprazole and Omeprazole.

Even more preferably, said drug is selected from the group comprising or, alternatively, consisting of:
- H2 receptor inhibitors, preferably cimetidine, famotidine, nizatidine or ranitidine;
- proton pump inhibitors, preferably selected from the group comprising Lansoprazole, Esomeprazole, Rabeprazole, Pantoprazole and Omeprazole.

In a preferred embodiment, the composition for use according to the present invention is a pharmaceutical composition comprising the bacteria set forth above, said bacteria being in association with a drug indicated for reducing or treating gastric hyperacidity, as specified above. Advantageously, the drug is a proton pump inhibitor selected from the group comprising Lansoprazole, Esomeprazole, Rabeprazole, Pantoprazole and Omeprazole. Both the bacteria and the drug are intimately present in the same composition.

For example, the bacteria and the drug are present together in a tablet, lozenge or granulate in a pharmaceutical form suitable for oral administration.

It is essential that the bacteria and the drug be administered simultaneously and act simultaneously, since it is necessary to restore the barrier effect removed by the proton pump inhibitors (PPI) thanks to the action of the probiotic bacteria of the present invention, which produce bacteriocins and are capable of colonizing the stomach thanks to the fact that the proton pump inhibitors have raised the pH to a value of about 4.5-5.0.

In another preferred embodiment, the composition for use according to the present invention is in the form of a medical device. In this case the bacteria are present in a composition suitable for oral administration, such as, for example, a tablet, lozenge or granulate and, separately, the drug indicated for reducing or treating gastric hyperacidity, as specified above, is present in another composition suitable for oral administration. Advantageously, the drug is a proton pump inhibitor selected from the group comprising Lansoprazole, Esomeprazole, Rabeprazole, Pantoprazole and Omeprazole.

Therefore, for example, two tablets are administered, one containing the bacteria and the other containing the drug.

In any case, the two tablets must be administered simultaneously, given that it is necessary for the bacteria to act simultaneously with the proton pump inhibitors.

In the case of a medical device as well, it is essential that the bacteria and the drug be administered within a short space of time from each other, since it is necessary to restore the barrier effect removed by the proton pump inhibitors (PPI) thanks to the action of the bacteria, which produce bacteriocins and are capable of colonizing the intestine thanks to the fact that the proton pump inhibitors have raised the pH to a value of about 4.5-5.0.

The Applicant has found that the bacteria selected and listed in Table 1 or Table 2 or in the preferred embodiments mentioned above, are capable of colonizing the stomach at a pH value of around 5 in such a way as to restore the barrier effect reduced or eliminated by the rise in the pH level resulting from the action of the drugs indicated for reducing or treating gastric hyperacidity, such as, for example, a proton pump inhibitor drug selected from the group comprising Lansoprazole, Esomeprazole, Rabeprazole, Pantoprazole and Omeprazole.

In a preferred embodiment, the composition containing the probiotic bacterial strains of the present invention - said strains being capable of producing specific bacteriocins - is also a useful adjuvant to treatments aimed at definitively eliminating *Helicobacter pylori* and avoiding recurrences thereof.

Therefore, the subject matter of the present invention is a composition comprising at least one strain of bacteria listed above, for use in the preventive and/or curative treatment of infections, disorders or diseases caused by the presence of *Helicobacter pylori,* in particular in the preventive and/or curative treatment of relapses of infections caused by *Helicobacter pylori.*

In the broadest sense of the term, antibiotics are defined as molecular species which are produced by an organism and are active against the growth of other organisms. In practice, however, antibiotics are generally considered secondary metabolites that are active at low concentrations in blocking the growth of microorganisms. Secondary products of metabolism such as organic acids, ammonia and hydrogen peroxide are not to be included in the category of antibiotics.

Antibiotics are molecules, also peptide molecules (penicillin), produced by multi-enzymatic systems whose biosynthesis is not blocked by protein synthesis inhibitors. Bacteriocins, on the other hand, are products of ribosomal synthesis.

Bacteriocins are peptide molecules synthesized by ribosomes which can also be associated with lipids or carbohydrates. Although some bacteriocins produced by Gram-positive bacteria *(Lactobacillus, Lactococcus)* possess inhibition spectra limited to some strains belonging to the same species as the producer microorganism, most of them show a broad spectrum of action against various bacterial species, both Gram-positive and Gram-negative. The present classification of bacteriocins is based both on their chemical nature and spectrum of action.

## Claims

1. A pharmaceutical or food composition or a supplement comprising four strains selected from the group consisting of:
*1. Lactobacillus pentosus* LPS01 DSM 21980
*2. Lactobacillus plantarum* LP01 LMG P-21021
3. *Lactobacillus plantarum* LP02 LMG P-21020
4. *Lactobacillus plantarum* LP03 LMG P-21022
5. *Lactobacillus plantarum* LP04 LMG P-21023
6. *Lactobacillus rhamnosus* LR06 DSM 21981
*7. Lactobacillus delbrueckii* LDD 01 (MB386) DSMZ 20074 DSM 22106.
*8. Bifidobacterium longum* B1975 DSM 24709
*9. Bifidobacterium breve* B2274 DSM 24707
10. *Bifidobacterium breve* B632 DSM 24706
11. *Bifidobacterium breve* B7840 DSM 24708
*12. Bifidobacterium longum* PCB 133 DSM 24691
13. *Bifidobacterium longum* BL06 DSM 24689,
which are capable of colonizing the stomach at a pH value comprised from 4.5 to 5 and of producing bacteriocins and/or hydrogen peroxide for use in the treatment of subjects who take drugs to reduce or treat gastric hyperacidity, wherein said pharmaceutical or food composition or supplement is capable of restoring the gastric barrier function.

2. The pharmaceutical or food composition or a supplement for use according to claim 1, wherein the pharmaceutical or food composition or supplement comprises four strains selected from the ones indicated in claim 1, in association with the strain *Lactobacillus fermentum* LF 09 DSM 18298 and/or the strain *Lactococcus lactis* NS 01 DSM 19072.

3. The pharmaceutical or food composition or supplement for use according to claim 1, wherein said composition comprises four strains selected from the ones indicated in claim 1, in association with at least one strain selected from the group consisting of:
a. *Lactobacillus reuteri* LRE 01 DSM 23877
b. *Lactobacillus reuteri* LRE 02 DSM 23878
c. *Lactobacillus reuteri* LRE 03 DSM 23879
d. *Lactobacillus reuteri* LRE 04 DSM 23880.

4. The pharmaceutical or food composition or supplement for use according to claim 1, wherein said drugs are selected from the group consisting of:
- H2 receptor inhibitors;
- prostaglandins;
- gastric mucosa protectors;
- muscarinic antagonists or parasympatholytic agents;
- antacids; and
- proton pump inhibitors.

5. The pharmaceutical or food composition or supplement for use according to claim 1, wherein said drugs are selected from the group consisting of:
- cimetidine, famotidine, nizatidine or ranitidine;
- misoprostol;
- bismuth salts or sucralfate;
- pirenzepine or pipenzolate;
- sodium bicarbonate, aluminium hydroxide, magnesium hydroxide; and
- Lansoprazole, Esomeprazole, Rabeprazole, Pantoprazole and Omeprazole.

6. The pharmaceutical or food composition or supplement for use according to claims 4 or 5, wherein said drugs are selected from the group consisting of:
- H2 receptor inhibitors;
- muscarinic antagonists or parasympatholytic agents;
- antacids; and
- proton pump inhibitors.

7. The pharmaceutical or food composition or supplement for use according to claim 6, wherein said drugs are selected from the group consisting of:
- cimetidine, famotidine, nizatidine or ranitidine;
- pirenzepine or pipenzolate;
- sodium bicarbonate, aluminium hydroxide, magnesium hydroxide;
- Lansoprazole, Esomeprazole, Rabeprazole, Pantoprazole and Omeprazole.

8. The pharmaceutical or food composition or supplement for use according to claim 7, wherein said drugs are selected from the group consisting of:
- cimetidine, famotidine, nizatidine or ranitidine; and
- Lansoprazole, Esomeprazole, Rabeprazole, Pantoprazole and Omeprazole.

9. The pharmaceutical or food composition or supplement for use according to any one of claims 1 to 8, wherein said composition is used in the treatment of subjects who take drugs to reduce or treat dyspepsia, gastroduodenal ulcer, gastric ulcer, peptic ulcer, duodenal ulcer, gastritis caused by *Helicobacter pylori* and gastroesophageal reflux disease.

10. A pharmaceutical composition comprising at least one bacterial strain according to any one of claims 1-3 and a drug according to any one of claims 4-9 for use in the treatment of subjects who take drugs to reduce or treat gastric hyperacidity, dyspepsia, gastroduodenal ulcer, gastric ulcer, peptic ulcer, duodenal ulcer, gastritis caused by *Helicobacter pylori* and gastroesophageal reflux disease.

11. The composition for use according to claim 10, wherein said bacterial strains and said drug are present together in a pharmaceutical form for oral use.

12. The composition according to any one of claims 1-11, for use in the preventive and/or curative treatment of infections, disorders or diseases caused by the presence of *Helicobacter pylori,* preferably in the preventive and/or curative treatment of relapses of infections caused by *Helicobacter pylori.*

## Patentansprüche

1. Arzneimittel- oder Lebensmittelzusammensetzung oder Ergänzungsmittel, umfassend vier Stämme, ausgewählt aus der Gruppe bestehend aus:
1. *Lactobacillus pentosus* LPS01 DSM 21980
2. *Lactobacillus plantarum* LP01 LMG P-21021
3. *Lactobacillus plantarum* LP02 LMG P-21020
4. *Lactobacillus plantarum* LP03 LMG P-21022
5. *Lactobacillus plantarum* LP04 LMG P-21023
6. *Lactobacillus rhamnosus* LR06 DSM 21981
7. *Lactobacillus delbrueckii* LDD 01 (MB386) DSMZ 20074 DSM 22106.
8. *Bifidobacterium longum* B1975 DSM 24709
9. *Bifidobacterium breve* B2274 DSM 24707
10. *Bifidobacterium breve* B632 DSM 24706
11. *Bifidobacterium breve* B7840 DSM 24708
12. *Bifidobacterium longum* PCB 133 DSM 24691
13. *Bifidobacterium longum* BL06 DSM 24689,
die in der Lage sind, den Magen bei einem pH-Wert im Bereich von 4,5 bis 5 zu besiedeln und Bacteriocine und/oder Wasserstoffperoxid zu produzieren, zur Verwendung bei der Behandlung von Patienten, die Arzneimittel einnehmen, um ihre gastrische Hyperazidität zu reduzieren oder zu behandeln, wobei diese Arzneimittel- oder Lebensmittelzusammensetzung oder dieses Ergänzungsmittel imstande ist, die gastrische Barrierefunktion wiederherzustellen.

2. Arzneimittel- oder Lebensmittelzusammensetzung oder Ergänzungsmittel zur Verwendung gemäß Anspruch 1, wobei die Arzneimittel- oder Lebensmittelzusammensetzung oder das Ergänzungsmittel vier Stämme, die aus denen von Anspruch 1 ausgewählt werden, in Verbindung mit dem Stamm *Lactobacillus fermentum* LF 09 DSM 18298 und/oder dem Stamm *Lactococcus lactis* NS 01 DSM 19072 umfasst.

3. Arzneimittel- oder Lebensmittelzusammensetzung oder Ergänzungsmittel zur Verwendung gemäß Anspruch 1, wobei die Zusammensetzung vier Stämme, ausgewählt aus denen von Anspruch 1, in Verbindung mit wenigstens einem Stamm, ausgewählt aus der Gruppe bestehend aus:
a. *Lactobacillus reuteri* LRE 01 DSM 23877
b. *Lactobacillus reuteri* LRE 02 DSM 23878
c. *Lactobacillus reuteri* LRE 03 DSM 23879
d. *Lactobacillus reuteri* LRE 04 DSM 23880
umfasst.

4. Arzneimittel- oder Lebensmittelzusammensetzung oder Ergänzungsmittel zur Verwendung gemäß Anspruch 1, wobei die Arzneimittel ausgewählt werden aus der Gruppe bestehend aus:
- H2 Rezeptor-Inhibitoren;
- Prostaglandinen;
- Magenschleimhaut schützenden Mitteln;
- Muscarinantagonisten oder parasympatholytischen Mitteln;
- Antazida; und
- Protonenpumpeninhibitoren.

5. Arzneimittel- oder Lebensmittelzusammensetzung oder Ergänzungsmittel zur Verwendung gemäß Anspruch 1, wobei die Wirkstoffe ausgewählt werden aus der Gruppe bestehend aus:
- Cimetidin, Famotidin, Nizatidin or Ranitidin;
- Misoprostol;
- Wismuthsalzen oder Sucralfat;
- Pirenzepin oder Pipenzolat;
- Natriumbicarbonat, Aluminiumhydroxid, Magnesiumhydroxid; und
- Lansoprazol, Esomeprazol, Rabeprazol, Pantoprazol und
Omeprazol.

6. Arzneimittel- oder Lebensmittelzusammensetzung oder Ergänzungsmittel zur Verwendung gemäß den Ansprüchen 4 oder 5, wobei die Wirkstoffe aus der Gruppe bestehend aus:
- H2 Rezeptor-Inhibitoren;
- Muscarinantagonisten oder parasympatholytischen Mitteln;
- Antazida; und
- Protonenpumpeninhibitoren
ausgewählt werden.

7. Arzneimittel- oder Lebensmittelzusammensetzung oder Ergänzungsmittel zur Verwendung gemäß Anspruch 6, wobei die Wirkstoffe aus der Gruppe bestehend aus:
- Cimetidin, Famotidin, Nizatidin or Ranitidin;
- Pirenzepin oder Pipenzolat;
- Natriumbicarbonat, Aluminiumhydroxid, Magnesiumhydroxid; und
- Lansoprazol, Esomeprazol, Rabeprazol, Pantoprazol und
Omeprazol
ausgewählt werden.

8. Arzneimittel- oder Lebensmittelzusammensetzung oder Ergänzungsmittel zur Verwendung gemäß Anspruch 7, wobei die Wirkstoffe aus der Gruppe bestehend aus:
- Cimetidin, Famotidin, Nizatidin or Ranitidin;
- Lansoprazol, Esomeprazol, Rabeprazol, Pantoprazol und
Omeprazol
ausgewählt werden.

9. Arzneimittel- oder Lebensmittelzusammensetzung oder Ergänzungsmittel zur Verwendung gemäß einem der Ansprüche 1 bis 8, wobei die Zusammensetzung bei der Behandlung von Patienten verwendet wird, die Arzneimittel einnehmen, um Dyspepsie, Magendarmgeschwüre, Magengeschwüre, peptische Geschwüre, Duodenalgeschwüre, von *Helicobacter pylori* verursachte Gastritis und gastroösophageale Refluxkrankheit zu verringern oder zu behandeln.

10. Arzneimittelzusammensetzung, umfassend wenigstens einen Bakterienstamm gemäß einem der Ansprüche 1 bis 3 und einen Wirkstoff gemäß einem der Ansprüche 4 bis 9 zur Verwendung bei der Behandlung von Patienten, die Arzneimittel einnehmen, um Dyspepsie, Magendarmgeschwüre, Magengeschwüre, peptische Geschwüre, Duodenalgeschwüre, von *Helicobacter pylori* verursachte Gastritis und gastroösophageale Refluxkrankheit zu verringern oder zu behandeln.

11. Zusammensetzung zur Verwendung gemäß Anspruch 10, wobei die Bakterienstämme und der Wirkstoff zusammen in einer pharmazeutischen Form zur oralen Verwendung vorliegen.

12. Zusammensetzung gemäß einem der Ansprüche 1 bis 11 zur Verwendung bei der präventiven und/oder curativen Behandlung von Infektionen, Störungen oder Krankheiten, die durch *Helicobacter pylori* verursacht werden, vorzugsweise bei der präventiven und/oder curativen Behandlung von relapsierenden Infektionen, die von *Helicobacter pylori* verursacht werden.

## Revendications

1. Composition pharmaceutique ou alimentaire ou complément comprenant quatre souches choisies dans le groupe constitué de :
1. *Lactobacillus pentosus* LPS01 DSM 21980
2. *Lactobacillus plantarum* LP01 LMG P-21021
3. *Lactobacillus plantarum* LP02 LMG P-21020
4. *Lactobacillus plantarum* LP03 LMG P-21022
5. *Lactobacillus plantarum* LP04 LMG P-21023
6. *Lactobacillus rhamnosus* LR06 DSM 21981
7. *Lactobacillus delbrueckii* LDD 01 (MB386) DSMZ 20074 DSM 22106
*8. Bifidobacterium longum* B1975 DSM 24709
*9. Bifidobacterium breve* B2274 DSM 24707
10. *Bifidobacterium breve* B632 DSM 24706
11. *Bifidobacterium breve* B7840 DSM 24708
12. *Bifidobacterium longum* PCB 133 DSM 24691
13. *Bifidobacterium longum* BL06 DSM 24689,
qui sont capables de coloniser l'estomac à une valeur de pH comprise entre 4,5 et 5 et de produire des bactériocines et/ou du peroxyde d'hydrogène, destinée à une utilisation dans le traitement de sujets qui prennent des médicaments pour réduire ou traiter une hyperacidité gastrique, où ladite composition pharmaceutique ou alimentaire ou ledit complément est capable de restaurer la fonction de la barrière gastrique.

2. Composition pharmaceutique ou alimentaire ou complément destiné à une utilisation selon la revendication 1, où la composition pharmaceutique ou alimentaire ou le complément comprend quatre souches choisies parmi celles indiquées dans la revendication 1, en association avec la souche *Lactobacillus fermentum* LF 09 DSM 18298 et/ou la souche *Lactococcus lactis* NS 01 DSM 19072.

3. Composition pharmaceutique ou alimentaire ou complément destiné à une utilisation selon la revendication 1, où ladite composition comprend quatre souches choisies parmi celles indiquées dans la revendication 1, en association avec au moins une souche choisie dans le groupe constitué de :
a. *Lactobacillus reuteri* LRE 01 DSM 23877
b. *Lactobacillus reuteri* LRE 02 DSM 23878
c. *Lactobacillus reuteri* LRE 03 DSM 23879
d. *Lactobacillus reuteri* LRE 04 DSM 23880.

4. Composition pharmaceutique ou alimentaire ou complément destiné à une utilisation selon la revendication 1, où lesdits médicaments sont choisis dans le groupe constitué de :
- inhibiteurs des récepteurs H2 ;
- prostaglandines ;
- protecteurs de la muqueuse gastrique ;
- antagonistes muscariniques ou agents parasympatholytiques ;
- antiacides ; et
- inhibiteurs de la pompe à protons.

5. Composition pharmaceutique ou alimentaire ou complément destiné à une utilisation selon la revendication 1, où lesdits médicaments sont choisis dans le groupe constitué de :
- cimétidine, famotidine, nizatidine ou ranitidine ;
- misoprostol ;
- sels de bismuth ou sucralfate ;
- pirenzépine ou pipenzolate ;
- bicarbonate de sodium, hydroxyde d'aluminium, hydroxyde de magnésium ; et
- lansoprazole, ésoméprazole, rabéprazole, pantoprazole et oméprazole.

6. Composition pharmaceutique ou alimentaire ou complément destiné à une utilisation selon les revendications 4 ou 5, où lesdits médicaments sont choisis dans le groupe constitué de :
- inhibiteurs des récepteurs H2 ;
- antagonistes muscariniques ou agents parasympatholytiques ;
- antiacides ; et
- inhibiteurs de la pompe à protons.

7. Composition pharmaceutique ou alimentaire ou complément destiné à une utilisation selon la revendication 6, où lesdits médicaments sont choisis dans le groupe constitué de :
- cimétidine, famotidine, nizatidine ou ranitidine ;
- pirenzépine ou pipenzolate ;
- bicarbonate de sodium, hydroxyde d'aluminium, hydroxyde de magnésium ;
- lansoprazole, ésoméprazole, rabéprazole, pantoprazole et oméprazole.

8. Composition pharmaceutique ou alimentaire ou complément destiné à une utilisation selon la revendication 7, où lesdits médicaments sont choisis dans le groupe constitué de :
- cimétidine, famotidine, nizatidine ou ranitidine ; et
- lansoprazole, ésoméprazole, rabéprazole, pantoprazole et oméprazole.

9. Composition pharmaceutique ou alimentaire ou complément destiné à une utilisation selon l'une quelconque des revendications 1 à 8, où ladite composition est utilisée dans le traitement de sujets qui prennent des médicaments pour réduire ou traiter une dyspepsie, un ulcère gastroduodénal, un ulcère gastrique, un ulcère peptique, un ulcère duodénal, une gastrique provoquée par *Helicobacter pylori* ou la maladie du reflux gastro-oesophagien.

10. Composition pharmaceutique comprenant au moins une souche bactérienne selon l'une quelconque des revendications 1 à 3 et un médicament selon l'une quelconque des revendications 4 à 9 destinée à une utilisation dans le traitement de sujets qui prennent des médicaments pour réduire ou traiter une dyspepsie, un ulcère gastroduodénal, un ulcère gastrique, un ulcère peptique, un ulcère duodénal, une gastrique provoquée par *Helicobacter pylori* ou la maladie du reflux gastrooesophagien.

11. Composition destinée à une utilisation selon la revendication 10, dans laquelle lesdites souches bactériennes et ledit médicament sont présents ensemble dans une forme pharmaceutique pour une utilisation orale.

12. Composition selon l'une quelconque des revendications 1 à 11, destinée à une utilisation dans le traitement préventif et/ou curatif d'infections, de troubles ou de maladies provoqués par la présence *d'Helicobacter pylori,* de préférence dans le traitement préventif et/ou curatif de rechutes d'infections provoquées par *Helicobacter pylori.*
